# FASCICULE DE BREVET EUROPEEN

(11) **EP 1 244 400 B1**
(45) Date de publication et mention de la délivrance du brevet: **02.03.2011**
(21) Numéro de dépôt: 01903854.6
(22) Date de dépôt: 02.01.2001
(51) Int. Cl.: A61F 11/04, A61B 5/00, H04R 25/00

(54) **DISPOSITIF DE MESURE OU DE RECUEIL DE POTENTIELS EVOQUES AUDITIFS**
VORRICHTUNG ZUR MESSUNG DES AUDITIVEN EVOZIERTEN POTENTIALS
DEVICE FOR MEASURING OR COLLECTING AUDITORY EVOKED POTENTIALS

(30) Priorité: 05.01.2000 FR 0000089; 27.01.2000 FR 0001126; 02.02.2000 US 496691
(43) Date de publication de la demande: 02.10.2002
(73) Titulaire: Battmer, Rolf-Dieter, 30539 Hannover (DE); Charvin, Guy, 06600 Antibes (FR); Lenarz, Thomas H.R., 30559 Hannover (DE)
(72) Inventeur: Battmer, Rolf-Dieter, 30539 Hannover (DE); Charvin, Guy, 06600 Antibes (FR); Lenarz, Thomas H.R., 30559 Hannover (DE)
(74) Mandataire: Bentz, Jean-Paul
(86) Numéro de dépôt international: PCT/FR2001/000009
(87) Numéro de publication internationale: WO 2001/050992

(56) Documents cités:
- WO-A-97/09863
- WO-A-97/32629
- WO-A-97/48447
- US-A- 5 755 747
- US-A- 5 758 651

## Description

La présente invention concerne un dispositif pour recueillir des signaux biologiques électriques, plus précisément des potentiels évoqués auditifs générés par une stimulation acoustique et/ou électrique et/ou mécanique de la cochlée, ou d'une partie du système auditif de l'homme ou de l'animal.

Les mesures de potentiels évoqués auditifs se font traditionnellement avec des dispositifs extracorporels utilisant des électrodes de surface appliquées sur la peau du patient. Ces équipements recueillent des potentiels évoqués auditifs à champ lointain, qui se décomposent principalement en:
- des potentiels évoqués auditifs précoces (appelés communément ABR). Ces potentiels sont composés normalement de sept ondes ("Les potentiels évoqués auditifs", J.M. GUERIT, 1993, Les potentiels évoqués pages 87 à 99, MASSON, PARIS), l'onde 1 étant principalement générée par les ramifications du nerf auditif le long des cellules ciliées internes ;
- des potentiels évoqués auditifs à latence moyenne (appelés AMLR) ;
- des potentiels évoqués auditifs tardifs (appelés ALCR) ;
- des potentiels évoqués cognitifs (appelés ACR).

Ces dispositifs extracorporels de recueil de potentiels auditifs demandent beaucoup de minutie et de temps pour leur mise en oeuvre. Par ailleurs, en terme de sécurité ils nécessitent d'avoir une parfaite isolation galvanique entre la machine et la personne testée. Les résultats obtenus ne représentent pas toujours fidèlement l'activité auditive réelle car ces équipements standards sont notamment trop sensibles aux bruits électriques parasites : artefacts de stimulation, les 50/60 Hz du secteur électrique, les interférences hautes fréquences, etc... De même, ils sont perturbés par les activités biologiques non auditives. Par ailleurs, la reproductibilité des résultats est altérée par la fluctuation de la résistivité de la peau et par des dérives d'impédance dues notamment aux électrodes mobiles qui peuvent bouger pendant le test et surtout être positionnées différemment d'un examen à l'autre. Pour limiter une partie de ces inconvénients on peut avoir recours à une anesthésie, notamment lors de tests sur les enfants, ce qui entraine d'autres inconvénients non négligeables.

Il existe aussi des dispositifs implantés de recueil de potentiels évoqués auditifs tels que décrits dans le brevet US 5,758,651 ou la demande WO 97/48447, qui mesurent l'activité de la cochlée suite à une stimulation électrique de cette dernière.

Le document US 5,758,651 décrit un système de télémesure pour une prothèse auditive, en particulier pour un implant cochléaire. Le système comporte une pluralité d'électrodes utilisées pour stimuler le nerf auditif et pour détecter les potentiels évoqués ; selon l'exemple décrit dans ce document, on mesure la différence de potentiel entre une électrode intracochléaire et une électrode extracochléaire ; cette différence de potentiel est appliquée à l'entrée d'un amplificateur désactivable ("blanking amplifier") et de gain réglable de 40 à 80 db ; selon une variante, ce document propose d'utiliser un amplificateur différentiel.

Le document WO 97/48447 décrit un système de réglage intégré à un implant de stimulation cochléaire ; ce système utilise deux moyens de mesure de la perception auditive par le patient : d'une part une électrode intracochléaire de mesure de potentiel évoqué, et d'autre part une électrode (ou transducteur) de mesure de l'activité de l'un des deux muscles ("stapedius" et "tensor tympani") de l'oreille moyenne.

Le document Carolyn J. Brown et al. (J. AcousT Soc AM, Vol. 88, n° 3 1385-1391, septembre 1990, pages 1385 à 1391) décrit une méthode de mesure des potentiels évoqués dans laquelle on utilise d'une part une des six électrodes intracochléaires de l'implant de stimulation, et d'autre part une électrode disposée sur le muscle temporal.

Ces dispositifs recueillent uniquement un potentiel évoqué auditif à champ proche, du fait qu'une des électrodes de recueil est immergée dans la cochlée et est la plus proche possible de l'activité à mesurer. Ce potentiel à champ proche, qui représente l'excitation initiale du système auditif, est communément appelé « Evoked Action Potential » (EAP). Il ne donne pas d'information sur les autres niveaux du système auditif : noyau cochléaire, coliculus inférieur, aires auditives primaires et secondaires, aires associatives. Par ailleurs, l'EAP ne peut pas être enregistré directement du fait des artefacts engendrés par une stimulation électrique trop proche des électrodes de recueil. Afin d'obtenir un EAP significatif, il est nécessaire d'utiliser des techniques dites « soustractives » permettant d'extraire l'artefact et de ne retenir que l'EAP. Ces techniques ont été notamment décrites dans le document Carolyn J. Brown et al. suscité.

Le dispositif selon l'invention permet de remédier aux inconvénients des dispositifs de recueils de potentiels évoqués existants.

Selon un premier aspect, l'invention consiste en un tel dispositif qui comporte au moins deux électrodes de recueil extra-cochléaires, destinées à être implantées, lesquelles électrodes de recueil sont reliées aux bornes d'entrée (positive et négative) d'un amplificateur différentiel intégré dans un boîtier, lui aussi destiné à être implanté ; en outre le boîtier comporte un système de transmission à travers la peau, avec ou sans traitement de signal, permettant à un dispositif externe de recevoir les informations relatives aux potentiels évoqués auditifs recueillis. Un tel dispositif permet d'enregistrer l'activité de chaque niveau du système auditif et d'évaluer périodiquement l'état fonctionnel du système auditif complet, de la cochlée jusqu'au cortex associatif ; la mise en oeuvre des mesures est quasi-immédiate, les artefacts quasi-inexistants, la constance dans le temps de la position et de l'impédance des électrodes de recueil limite l'imprécision des mesures et permet une bonne reproductibilité. Enfin, l'éloignement relatif des électrodes de recueil du site cochléaire permet des mesures précises et directes de tous les potentiels évoqués auditifs à champ lointain décrits ci-dessus : ABR, AMLR, ALCR, ACR. Par ailleurs, l'onde 1 de l'ABR permet, par équivalence (cf Burkard et al, J Acous Soc Am, 93 (4), pages 2069-2076, avril 1993), de mesurer la même activité que celle de l'EAP, et ce, sans devoir recourir à une technique « soustractive ». Le dispositif objet de l'invention permet donc l'exploration et l'évaluation de l'activité globale du système auditif avec possibilité d'enregistrement des résultats en une seule opération. Il permet aussi, lorsqu'il est couplé à un implant cochléaire, d'optimiser les réglages de ce dernier, avec rapidité et d'une manière « objective », notamment chez le jeune enfant, ce qu'aucun des dispositifs connus à ce jour ne permet.

L'invention repose en partie sur la constatation selon laquelle il est possible de mesurer de façon fiable les potentiels électriques résultant de l'activité neuronale du système auditif à l'aide d'au moins deux électrodes éloignées de la cochlée ; un avantage est la plus grande facilité d'implantation chirurgicale du dispositif ; un autre avantage est la moindre sensibilité du système de mesure aux artefacts de stimulation de la cochlée. Une particularité essentielle du procédé et du dispositif de mesure selon l'invention est que, du fait de la position externe (à la cochlée) des électrodes de mesure (recueil), les différences de potentiels mesurés témoignent de phénomènes physiques bien distincts de ceux dont témoignent les mesures utilisant une électrode intracochléaire.

Un autre avantage important de l'invention est qu'il est possible d'utiliser des électrodes de mesure plus grosses que celles pouvant être implantées dans la cochlée; il en résulte que l'impédance de contact entre l'électrode et le tissu est diminuée et peut être maintenue sensiblement constante dans le temps ; la précision et la répétabilité des mesures sont donc améliorées; par ailleurs, la taille de l'électrode peut permettre de lui associer une vis la traversant pour sa fixation dans un tissu osseux, améliorant d'une part la stabilité dans le temps de l'impédance, et pouvant contribuer à la fixation du boîtier de l'implant lui-même.

La technique de mesure selon l'invention est beaucoup moins sensible aux artefacts de stimulation électrique ; il est par conséquent possible de mesurer et d'étudier les potentiels évoqués dans la "fenêtre temporelle" d'environ 400 µs suivant la stimulation, contrairement aux systèmes décrits dans les documents ci-dessus ; en outre, le dispositif selon l'invention est simplifié car un amplificateur désactivable ("blanking amplifier") n'est pas nécessaire, et car il n'est pas non plus utile de prévoir la mise en court-circuit - ou en circuit ouvert - des électrodes.

Etant donné que l'amplitude des différences de potentiels détectées sont très faibles, en particulier de l'ordre de 0,1 à 1 microvolt, soit cent à mille fois inférieures à celles mesurées lorsqu'on place une électrode de mesure dans la cochlée, il est généralement prévu une troisième électrode de mesure qui sert de référence et est raccordée à la borne de masse de l'amplificateur différentiel: ceci permet d'augmenter notablement le rapport signal sur bruit en sortie.

Selon des modes préférés de réalisation :
- les électrodes de recueil, qui sont au minimum de deux, se situent de part et d'autre du boîtier-amplificateur-récepteur-émetteur implantable, dans une position sensiblement diamétralement opposée ; ceci permet, lors de l'implantation chirurgicale des électrodes, de les aligner facilement sur l'axe de la portion du système auditif associé, de manière à optimiser les amplitudes des signaux recueillis;
- les électrodes de recueil peuvent être mobiles par rapport au boîtier de manière à être positionnées d'une manière optimale en per-opératoire par le chirurgien, par exemple bien parallèles au tronc cérébral, correctement éloignées l'une de l'autre ; à cet effet, les électrodes sont de préférence montées à l'extrémité d'un support tel qu'un cathéter souple en silicone enveloppant le fil conducteur en liaison avec le boîtier;
- les électrodes de recueil peuvent être alternativement disposées sur un support en forme de plaque souple s'étendant autour du boitier-récepteur-émetteur ; ceci permet au praticien, après implantation chirurgicale, de choisir deux électrodes de recueil (parmi la pluralité d'électrodes implantées) formant le couple optimal pour l'activité à mesurer, grâce à un dispositif de multi-plexage incorporé au boîtier implanté ;
- l'électrode de référence de recueil est disposée à proximité ou sur le boîtier implantable.

Dans le cas où le dispositif implantable incorpore des moyens de stimulation électrique du système nerveux auditif:
- l'électrode de référence de stimulation peut être montée mobile par rapport au boîtier, de manière à être positionnée d'une manière optimale, à partir de tests effectués en per-opératoire, puis fixée durablement par le chirurgien ; cette optimisation peut consister à faire en sorte que la ligne du dipôle de stimulation « électrodes de stimulation/électrode de référence de stimulation » soit perpendiculaire à la ligne reliant les deux électrodes de recueil, afin de réduire l'artefact de stimulation ;
- il peut être prévu plusieurs électrodes fixes de référence de stimulation, reliées en aval à un dispositif de multiplexage intégré au boîtier : ceci permet au praticien, après implantation chirurgicale, de choisir l'électrode de référence de stimulation qui réduit au mieux l'artefact de stimulation ;
- il peut également être prévu plusieurs électrodes de recueil et plusieurs électrodes de référence de stimulation reliées à deux systèmes respectifs de multiplexage permettant d'optimiser les réglages après l'intervention chirurgicale, dans le but d'éviter des tests en per-opératoire.

Dans certains cas, l'électrode de référence de recueil peut être la même que l'électrode de référence de stimulation.

Le transfert vers l'extérieur des signaux physiologiques recueillis, notamment des potentiels évoqués auditifs, s'effectue, au niveau du boïtier-récepteur-émetteur implanté, par transmission sans fil (exemple: haute fréquence par modulation d'amplitude ou de fréquence).

Le dispositif «électrodes de recueil / électrode de référence de recueil / boitier-récepteur-émetteur implantable » peut être couplé ou intégré à un dispositif de stimulation acoustique, et/ou électrique, et/ou mécanique, extra-cochléaire, et/ou intra-cochléaire. Dans ce cas, il constitue un implant de stimulation équipé d'un dispositif de télémesure permettant d'évaluer ou de contrôler son action. A titre d'exemple, le dispositif objet de l'invention est:
- soit un implant cochléaire pour les surdités sévères ou profondes, comportant une ou des électrode(s) de stimulation intracochléaire(s), équipé d'un dispositif de recueil de potentiels évoqués auditifs permettant d'effectuer les réglages optimaux des seuils de détection, des seuils d'inconfort et de la fonction de compression acoustique électrique;
- soit un implant auditif pour les surdités sévères ou profondes comportant une ou des électrodes(s) de stimulation extra-cochléaire(s) ; un tel implant peut préférentiellement comporter une électrode de stimulation électrique fixable sur l'étrier ; il est équipé bien sûr du même dispositif de recueil des potentiels évoqués auditifs permettant les réglages optimaux ;
- soit un implant auditif pour les surdités moyennes ou sévères, comportant un dispositif de stimulation mécanique du système auditif tel qu'un vibreur, positionnable préférentiellement sur la fenêtre ovale de la cochlée, ou dans la mastoïde,
- soit un implant de neutralisation des acouphènes, équipé d'un dispositif de recueil des potentiels évoqués auditifs pour le réglage optimal de la stimulation électrique.

D'autres avantages et caractéristiques de l'invention seront compris au travers de la description suivante qui se réfère aux dessins annexés, qui illustrent sans aucun caractère limitatif des modes préférentiels de réalisation de l'invention.
La figure 1a représente un dispositif implantable selon un premier mode de réalisation.
La figure 1b représente un dispositif implantable selon un deuxième mode de réalisation.
La figure 2a illustre schématiquement le système de transmission transcutanée entre un dispositif implanté et un dispositif externe d'analyse de signaux.
La figure 2b illustre la connexion des électrodes à un amplificateur dont la sortie est connectée à un émetteur et à un module de traitement de signaux.
La figure 2c illustre une variante de la figure 2b, dans laquelle une pluralité d'électrodes de recueil telles que celles illustrées figure 1b sont connectées à l'amplificateur par deux multiplexeurs.
La figure 3 illustre quatre chronogrammes de mesure de tension selon quatre configurations respectives d'implantation des électrodes de recueil.
La figure 4 illustre trois chronogrammes de mesure de tension selon trois configurations respectives d'implantation des électrodes de recueil.
La figure 5 illustre deux variantes d'implantation des électrodes de stimulation, et deux chronogrammes de l'artefact correspondant respectivement à ces deux implantations.
La figure 6 illustre un chronogramme de mesure par des électrodes de surface, et un chronogramme résultant d'une moyenne de mesures effectuées avec le dispositif de la figure la.
La figure 7a représente un dispositif implantable selon un troisième mode de réalisation.
La figure 7b représente le dispositif d'orientation d'électrodes de la figure 7a.
La figure 8 représente un dispositif implantable selon un quatrième mode de réalisation.
La figure 9a illustre la connexion de plusieurs électrodes de référence Rri à l'amplificateur par l'intermédiaire d'un multiplexeur.
La figure 9b illustre la connexion de plusieurs électrodes de stimulation à un module de stimulation par deux démultiplexeurs.
La figure 10a représente un dispositif implantable selon un cinquième mode de réalisation.
La figure 10b représente à échelle agrandie le transducteur électromécanique de la figure 10a.

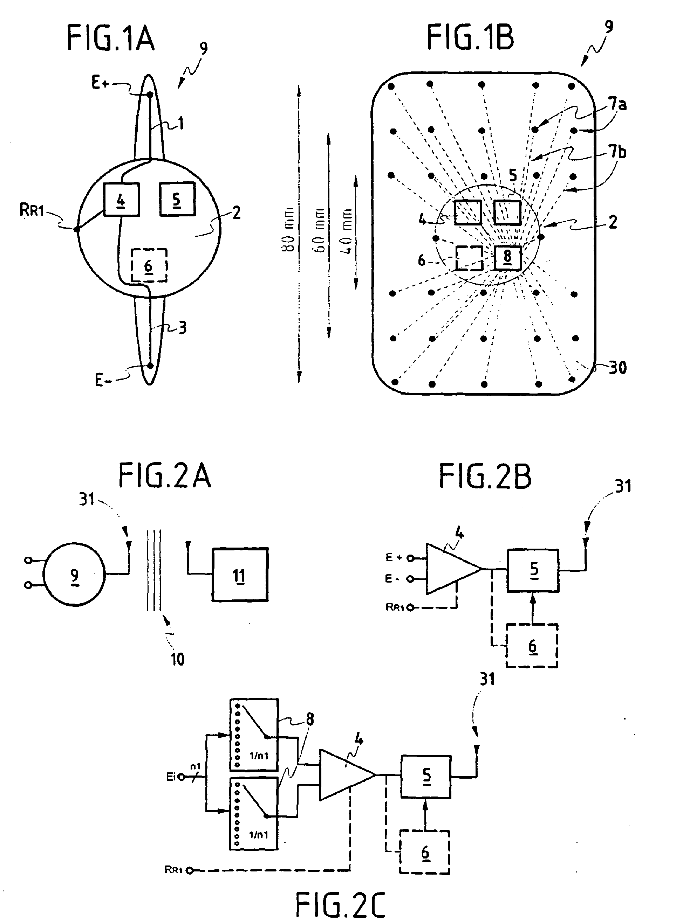

La figure 1a représente un dispositif implantable qui permet, d'une part de recueillir les signaux biologiques électriques caractéristiques des potentiels évoqués auditifs, et ce, via les électrodes de recueil positive (E +) et négative (E -) connectées respectivement par les fils 1 et 3 au module-amplificateur 4 contenu dans le boîtier 2 étanche, et permet d'autre part de transmettre à l'extérieur du corps lesdits signaux ou des signaux dérivés de ceux-ci, via le module-émetteur 5 contenu dans le boîtier 2, et ce, par radio-fréquence. La bobine ou l'antenne 31 du module émetteur 5 peut être indifféremment à l'intérieur ou à l'extérieur du boîtier 2. Le boîtier contient un module optionnel de traitement du signal 6. L'électrode dite de référence (RR1), ici fixée au boîtier, est utilisée pour permettre une amplification différentielle entre les deux électrodes de recueil. L'énergie nécessaire au fonctionnement de l'implant peut soit provenir d'une batterie ou pile implantable, soit être transmise, via une bobine extérieure, par induction électromagnétique.

Le dispositif représenté figure 1b permet, via une grille de trente deux électrodes 7a régulièrement espacées sur une plaque souple 30 et reliées par des fils 7b (indiqués en pointillés) à un module de multiplexage 8 contenu dans le boîtier 2, de sélectionner par commutation électronique les deux électrodes retenues comme formant un couple optimal pour recueillir l'activité physiologique que l'on souhaite mesurer, ces couples se distinguant par leur orientation et la distance des électrodes l'une par rapport à l'autre ; la distance entre les électrodes est préférentiellement comprise entre 40 mm et 80 mm (une distance d'environ 60 mm apparaissant comme optimale).

La figure 2a représente le principe de fonctionnement du dispositif. Le dispositif implantable 9, tel que décrit figure 1, est chargé de recueillir l'activité physiologique électrique suite à une stimulation du système auditif et la transmettre via la peau 10 du patient à une unité externe 11 chargée d'analyser cette activité. La transmission se fait de manière transcutanée et permet un isolement galvanique. La figure 2b représente le schéma général de fonctionnement dudit dispositif comportant deux électrodes E+ et E- de recueil ; la figure 2c représente un dispositif comportant un nombre n₁ de paires d'électrodes Ei de recueil connectées aux entrées de deux multiplexeurs 8 dont les sorties respectives sont raccordées aux entrées de l'amplificateur 4.

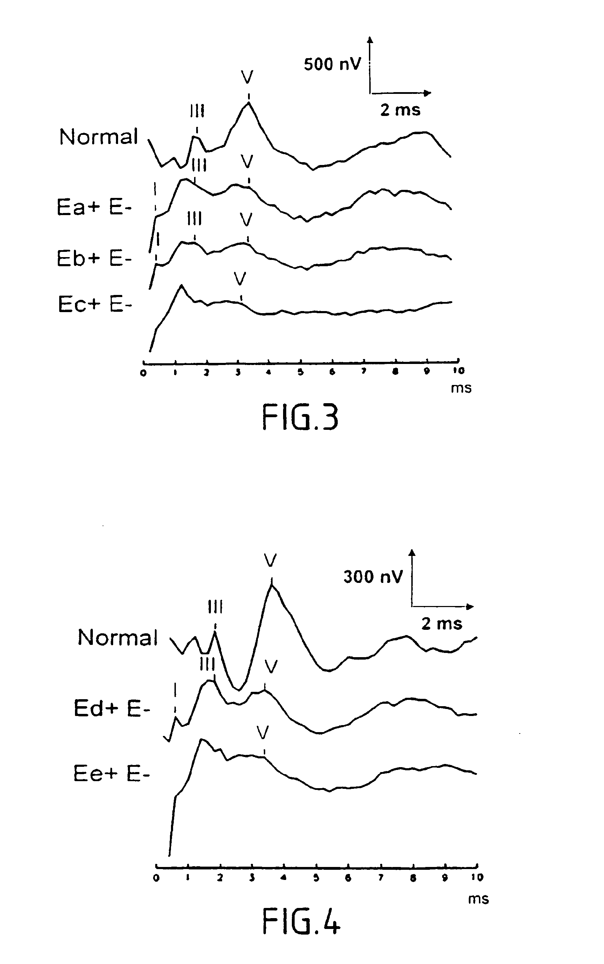

La figure 3 montre trois exemples de relevé de mesures (de tension en fonction du temps) obtenues à l'aide dudit dispositif. Ces courbes montrent que la distance qui sépare les deux électrodes de recueil influence de manière importante le recueil des caractéristiques des potentiels évoqués auditifs précoces (ABR). En comparaison avec un recueil standard extracorporel (courbe supérieure indiquée ici "normal"), à l'aide d'une électrode de surface positive sur le front, et d'une électrode de surface négative sur la mastoïde homolatérale, la qualité des recueils des ABR varie selon la distance qui sépare les deux électrodes de recueil implantées: les résultats obtenus avec deux électrodes Ea+, E- espacées de 60 mm sont d'une qualité nettement meilleure qu'avec deux électrodes Eb+, E-espacées de 45 mm, laquelle est nettement meilleure qu'avec deux électrodes Ec+, E- espacées de 30mm. Le dispositif selon l'invention comporte préférentiellement des électrodes de recueil espacées de plus de 30 mm l'une de l'autre.

La figure 4 représente d'autres relevés qui montrent que orientation des électrodes de recueil par rapport à l'activité que l'on veut mesurer, ici le tronc cérébral, influence le recueil des potentiels évoqués positifs. En comparaison avec un recueil standard extracorporel (indiqué ici "normal" ; une électrode de surface positive sur le front, une électrode de surface négative sur la mastoïde homolatérale) la qualité des recueils des PEAP décroît en fonction de l'orientation des deux électrodes de recueil implanté par rapport à l'activité à mesurer: les résultats obtenus avec deux électrodes Ed+, E- orientées parallèlement au tronc cérébral sont meilleurs que ceux obtenus avec deux électrodes Ee+, E- orientées à 28° du tronc cérébral. Le dispositif selon l'invention qui est adapté pour l'obtention des PEAPdoit être orienté pour que les électrodes de recueil soient le plus parallèle possible au tronc cérébral.

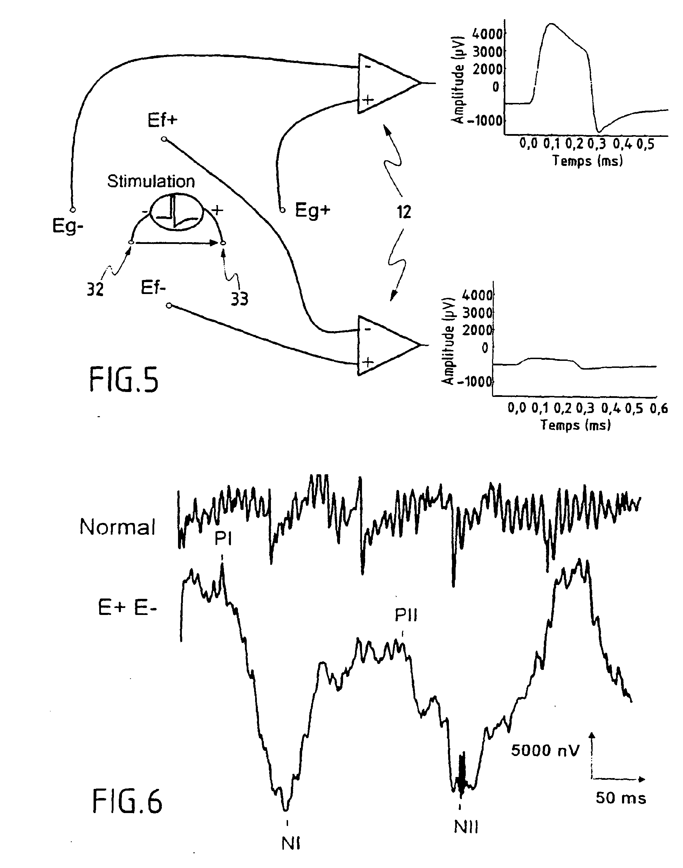

La figure 5 montre que, lors d'une stimulation électrique du système auditif, l'orientation des électrodes de recueil par rapport aux électrodes 32, 33 de stimulation influence le recueil de l'artefact de stimulation. Avec une position de recueil parallèle à la stimulation (Eg+Eg-) l'amplitude de l'artefact de stimulation sortant de l'amplificateur différentiel 12 est maximale. Avec une position de recueil perpendiculaire à la stimulation (Ef+Ef-) l'amplitude de l'artefact de stimulation sortant de l'amplificateur différentiel (12) est minimale. Le dispositif selon l'invention comporte préférentiellement des électrodes de recueil perpendiculaires au dipôle de stimulation équivalent.

La figure 6 représente un recueil de potentiels évoqués auditifs tardifs (ALCR) obtenus avec d'une part des électrodes de surface conventionnelles (référencé Normal sur le dessin), et d'autre part avec le dispositif décrit figure 1a (référencé E+E-), suite à une stimulation électrique intracochléaire.

La distance entre les électrodes + et - de l'amplificateur différentiel est d'environ 60 mm et semble un bon compromis pour l'atténuation maximum des artefacts et une implantation chirurgicale facile. 11 n'a suffit que d'un moyennage de 100 recueils pour obtenir ce ALCR de bonne qualité. Cela s'explique par le fait que l'amplitude des ondes NI, PI, NII, PII est de l'ordre de 25µV, soit 10 fois celles obtenues avec le dispositif de recueil à électrodes externes de surfaces (réf. "Les potentiels évoqués auditifs", J.M. GUERIT, 1993, Les potentiels évoqués pages 87 à 99, MASSON, PARIS)."

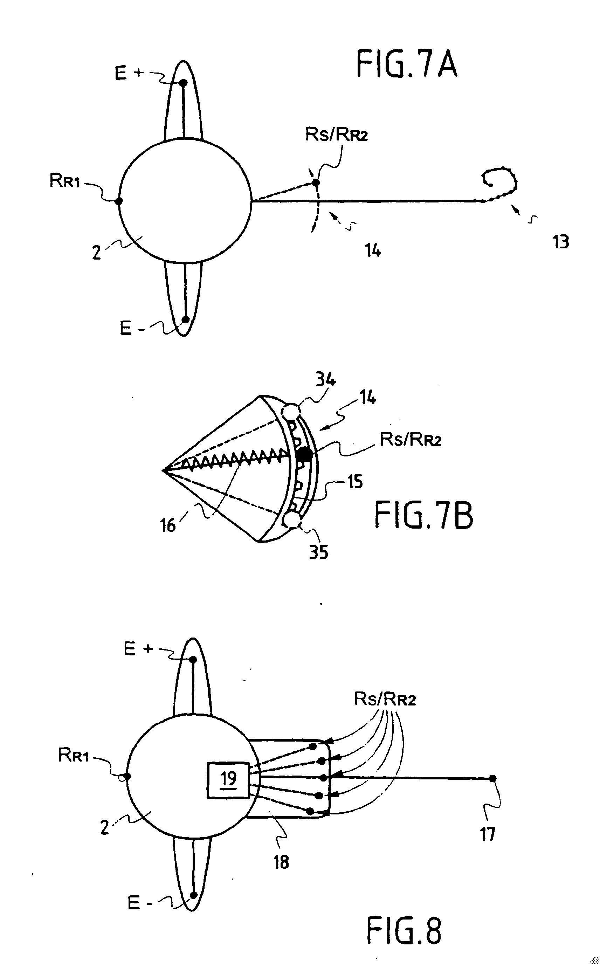

La figure 7a représente un implant cochléaire équipé d'un dispositif de télémesure des potentiels évoqués auditifs avec transmission des mesures par radio-fréquence. Cet implant permet, tout en stimulant la cochlée via l'électrode intracochléaire 13 (laquelle peut comporter une unique électrode ou plusieurs électrodes différenciées) et l'électrode de référence de stimulation RS de recueillir l'activité du système auditif via les électrodes E+ / E-. Dans le cas d'une amplification différentielle, l'électrode de référence peut être ou fixe (RR1) ou mobile (RR2). Selon un mode particulier de réalisation, l'électrode de référence de stimulation RS peut être utilisée comme électrode de référence de recueil RR2. Toute l'électronique nécessaire à stimuler, recueillir et transmettre se situe dans le boîtier étanche 2. La (ou les) bobine(s) ou antenne(s) de communication, et le cas échéant de transmission d'énergie peut (ou peuvent) être indifféremment à l'intérieur ou à l'extérieur du boîtier 2. Un dispositif 14 permet d'orienter le dipôle de stimulation en déplaçant l'électrode RS/RR2 ; ce dispositif 14 illustré figure 7b, selon un mode de réalisation de l'invention, comporte une électrode RS/RR2 qui est guidée par un système coulissant à différentes positions crantées 15. Un fil électrique 16 en spirale et siliconé souple permet le déplacement, sans contraintes mécaniques importantes, de l'électrode RS/RR2 entre deux positions extrêmes 34 et 35.

La figure 8 représente un dispositif de stimulation extracochléaire muni d'une ou plusieurs électrodes de stimulation 17, et intégrant un dispositif de télémesure des potentiels évoqués. Il permet en stimulant électriquement via l'électrode 17 (comportant une ou plusieurs électrode(s) différenciée(s)) et l'électrode de référence de stimulation RS de recueillir l'activité électrique du système auditif via les électrodes E+/E-. Selon un mode particulier de réalisation l'électrode de référence de stimulation RS peut être utilisée comme électrode de référence de recueil (ici RR2). Il est de plus équipé de plusieurs électrodes de référence de recueil et de référence de stimulation RS/RR2 disposées sur un support 18 et reliées par des fils à un module de commutation 19 situé dans le boîtier étanche 2 qui permet de choisir l'électrode RS/RR2. L'électrode dite de référence de recueil (RR1 ou RS/RR2) peut être utilisée pour permettre une amplification différentielle entre les deux électrodes de recueil. Toute l'électronique nécessaire à stimuler, recueillir et transmettre se situe dans le boîtier 2. La (ou les) bobine(s) ou antenne(s) de communication, et le cas échéant de transmission d'énergie, peut (ou peuvent) être indifféremment à l'intérieur ou à l'extérieur du boîtier 2.

Les implants décrits aux figures 7 est 8 peuvent être équipés soit d'une électrode unique RR1 fixe ou mobile, soit de plusieurs électrodes RR1 (et/ou RR2) gérées par un commutateur 19, soit d'une électrode unique RS/RR2 fixe ou préférentiellement mobile, ou soit de plusieurs électrodes RS/RR2 gérées par un commutateur 19. L'énergie nécessaire au fonctionnement de l'implant peut soit provenir d'une batterie ou une pile implantable, ou soit être transmise, via une bobine extérieure, par induction électromagnétique.

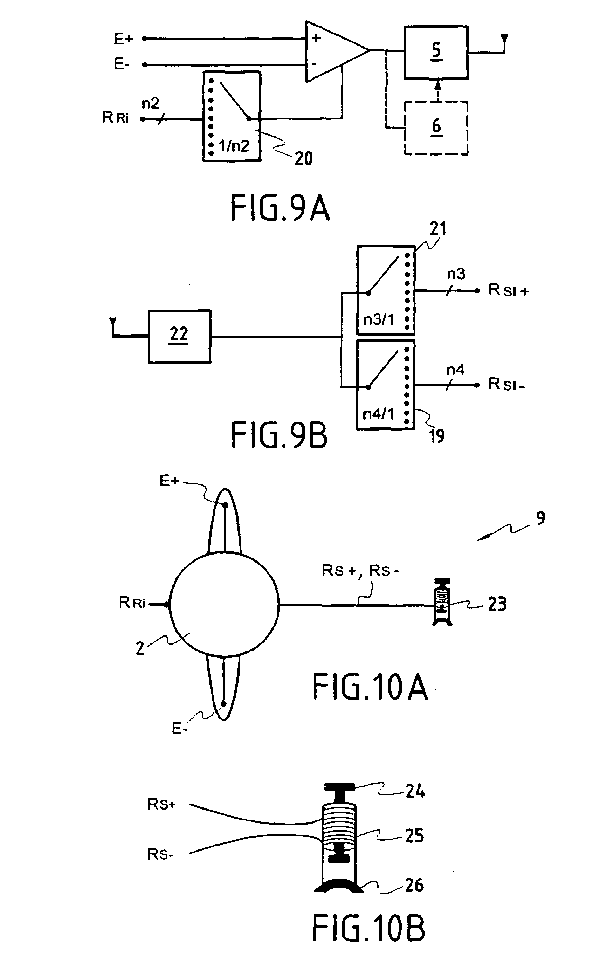

Les figures 9a et 9b représentent le schéma de fonctionnement d'un implant du type de ceux décrits dans les figures 7 et 8.

La figure 9a correspond à la partie "recueil" du dispositif. Le module 20 est un circuit de multiplexage qui permet de choisir l'électrode de référence parmi les n2 électrodes RRi disponibles.

La figure 9b correspond à la partie "stimulation" électrique du dispositif. Le module 21 est un circuit de démultiplexage qui va choisir l'électrode positive du stimulateur parmi les n3 électrodes Rsi+ disponibles.

Le module 19 est un circuit de démultiplexage qui va choisir l'électrode négative du stimulateur parmi les n4 électrodes Rsi- disponibles.

Le module 22 pilote les deux démultiplexeurs 19 et 21 en fonction de l'information qu'il reçoit de l'extérieur via une antenne.

La figure 10a représente un stimulateur mécanique de l'oreille moyenne équipé d'un dispositif de télémesure de potentiels évoqués auditifs. Cet implant permet, en stimulant par vibration mécanique la fenêtre ovale de la cochlée, de recueillir l'activité du système auditif via les électrodes E+/E-. Le vibreur 23 utilisé pour la stimulation mécanique est décrit figure 10b. Celui-ci, suite à une chirurgie très proche de celle pratiquée lors d'une pathologie appelé otostongiose, vient remplacer l'osselet appelé étrier. L'extrémité 24 vient se poser sur la fenêtre ovale à la place de la platine de l'étrier. Le transducteur 25, qui va transformer un signal électrique en énergie mécanique, peut être magnétique ou piézo-électrique. Sa fonction est de déplacer la partie 24 par rapport à la partie 26 en fonction d'un signal de commande provenant du boîtier 2 et transmis par les deux fils RS+ et RS-. Toute l'électronique nécessaire à stimuler, recueillir et transmettre, se situe dans le boîtier étanche 2. La ou les bobines(s) ou antenne(s) de communication, et le cas échéant de transmission d'énergie, peut ou peuvent être indifféremment à l'intérieur ou à l'extérieur du boîtier 2.

## Revendications

1. Dispositif implantable (9) de mesure ou de recueil de potentiels évoqués auditifs, comportant un dispositif (13, 17, 23) de stimulation pour stimuler le système auditif, au moins une paire d'électrodes de recueil extra-cochléaires (E⁺, E⁻, 7a) implantables dans le corps d'un patient ou d'un animal, pour détecter des signaux électriques résultant de l'activité neuronale du système auditif, et un module amplificateur (4) ayant des entrées connectées respectivement aux électrodes de recueil extra-cochléaires (E⁺, E⁻, 7a), pour produire des signaux représentatifs de l'activité neuronale du système auditif, à partir des signaux électriques détectés par les électrodes de recueil, extra-cochléaires **caractérisé en ce que** les électrodes (E⁺, E⁻, 7a) de recueil extra-cochléaires sont réparties sur un support (30) souple autour d'un boîtier (2) intégrant le module amplificateur (4).

2. Dispositif selon la revendication 1, dans lequel le module amplificateur (4) comprend un amplificateur différentiel (12) dont les entrées sont raccordées aux électrodes de recueil (E⁺, E⁻, 7a) et dont la masse est raccordée à une électrode (RR₁, RR₂) de référence de recueil.

3. Dispositif selon la revendication 1 ou 2, dans lequel les signaux électriques fournis au module amplificateur (4) par les électrodes de recueil (E⁺, E⁻, 7a) présentent une amplitude de l'ordre de 0,1 à 1 µV.

4. Dispositif selon l'une des revendications 1 à 3, dans lequel les électrodes (E⁺, E⁻, 7a) de recueil sont distantes de plus de 3 cm.

5. Dispositif selon l'une des revendications 1 à 4, dans lequel le boîtier (2) étanche intégrant le module amplificateur (4) comprend aussi un émetteur (5) pour la transmission sans contact à travers la peau (10) de signaux radio à une unité externe (11).

6. Dispositif selon l'une des revendications 1 à 5, dans lequel les électrodes (E⁺, E⁻, 7a) de recueil sont disposées de part et d'autre du boîtier (2) intégrant le module amplificateur (4), dans une position sensiblement diamétralement opposée, et sont montées à l'extrémité d'un support souple permettant leur positionnement optimal.

7. Dispositif selon l'une quelconque des revendications 1 à 6, dans lequel les électrodes de recueil sont perpendiculaires au dipôle de stimulation équivalent.

8. Dispositif selon l'une quelconque des revendications 1 à 7, dans lequel une électrode (RR₁) de référence de recueil est fixée sur le boîtier (2) dans lequel est intégré le module amplificateur (4).

9. Dispositif selon l'une quelconque des revendications 1 à 8, dans lequel une électrode (RR₂, RS/RR₂) de référence de recueil est montée mobile par rapport au boîtier (2) intégrant le module amplificateur (4).

10. Dispositif selon l'une quelconque des revendications 1 à 9, qui comporte au moins deux électrodes (RR2, RS/RR) de référence de recueil qui sont raccordées à un commutateur (19).

11. Dispositif selon l'une quelconque des revendications précédentes, dans lequel le dispositif de stimulation comprend au moins une électrode (13, 17) de stimulation pour stimuler électriquement le système auditif, ou dans lequel le dispositif de stimulation comporte au moins un vibreur (23) pour stimuler mécaniquement le système auditif.

12. Dispositif selon la revendication 11 dans lequel l'électrode (13) de stimulation est une électrode intra-cochléaire.

13. Dispositif selon la revendication 11 dans lequel l'électrode (13) de stimulation est une électrode extra-cochléaire.

14. Dispositif selon l'une quelconque des revendications 1 à 13 comportant au moins une électrode de référence de recueil (RR₂) couplée à au moins une électrode de référence de stimulation (RS).

15. Dispositif selon l'une quelconque des revendications précédentes, comprenant en outre au moins un multiplexeur (8) intégré dans le boîtier (2) et dans lequel ladite au moins une paire d'électrodes (E⁺, E⁻, 7a) de recueil est connectée au module amplificateur (4) par l'intermédiaire dudit au moins un multiplexeur (8).

## Claims

1. Implantable device (9) for measuring or picking up auditory evoked potentials, including a stimulation device (13, 17, 23) for stimulating the auditory system, at least one pair of extracochlear pickup electrodes (E⁺, E⁻, 7a) implantable in the body of a patient or an animal to detect electrical signals that result from neural activity of the auditory system, and an amplifier module (4) having inputs connected to respective extracochlear pickup electrodes (E⁺, E⁻, 7a) to produce signals representative of the neural activity of the auditory system on the basis of electrical signals detected by the extracochlear pickup electrodes, **characterized in that** the extracochlear pickup electrodes (E⁺, E⁻, 7a) are distributed on a flexible support (30) around a package (2) containing the amplifier module (4).

2. Device according to Claim 1, in which the amplifier module (4) comprises a differential amplifier (12) having inputs that are connected to the pickup electrodes (E⁺, E⁻, 7a) and a ground that is connected to a pickup reference electrode (RR₁, RR₂).

3. Device according to Claim 1 or Claim 2, in which the electrical signals supplied to the amplifier module (4) by the pickup electrodes (E⁺, E⁻, 7a) have an amplitude of the order of 0.1 to 1 µV.

4. Device according to one of Claims 1 to 3, in which the pickup electrodes (E⁺, E⁻, 7a) are spaced apart by more than 3 cm.

5. Device according to one of Claims 1 to 4, in which the sealed package (2) containing the amplifier module (4) also contains a transmitter (5) for contactless transmission through the skin (10) of radio signals to an external unit (11).

6. Device according to one of Claims 1 to 5, in which the pickup electrodes (E⁺, E⁻, 7a) are placed on either side of the package (2) containing the amplifier module (4) in substantially diametrically opposite positions and are mounted at the end of a flexible support enabling them to be positioned optimally.

7. Device according to one of Claims 1 to 6, in which the pickup electrodes are perpendicular to the equivalent stimulation dipole.

8. Device according to any one of Claims 1 to 7, in which a pickup reference electrode (RR₁) is fixed to the package (2) containing the amplifier module (4).

9. Device according to any one of Claims 1 to 8, in which a pickup reference electrode (RR₂, RS/RR₂) is movably mounted relative to the package (2) containing the amplifier module (4).

10. Device according to any one of Claims 1 to 9, which includes at least two pickup reference electrodes (RR2, RS/RR) which are connected to a switch (19).

11. Device according to any of the preceding claims, in which the stimulation device comprises at least one stimulation electrode (13, 17) for electrically stimulating the auditory system or in which the stimulation device includes at least one vibrator (23) for mechanically stimulating the auditory system.

12. Device according to Claim 11 in which the stimulation electrode (13) is an intracochlear electrode.

13. Device according to Claim 11 in which the stimulation electrode (13) is an extracochlear electrode.

14. Device according to any one of Claims 1 to 13 including at least one pickup reference electrode (RR₂) coupled to at least one stimulation reference electrode (RS).

15. Device according to any of the preceding claims, further comprising at least one multiplexer (8) incorporated in the package (2) and in which said at least one pair of pickup electrodes (E⁺, E⁻, 7a) is connected to the amplifier module (4) via said at least one multiplexer (8).

## Patentansprüche

1. Implantierbare Vorrichtung (9) zum Messen oder Sammeln von akustisch evozierten Potentialen, mit einer Stimulationseinrichtung (13, 17, 23) zum Stimulieren des Hörsystems, wenigstens einem Paar extrakochleare Empfangselektroden (E⁺, E⁻, 7a), die in den Körper eines Patienten oder eines Tieres implantierbar sind, um elektrische Signale zu erfassen, die sich aus einer neuronalen Aktivität des Hörsystems ergeben, und einem Verstärkermodul (4), das Eingänge aufweist, die jeweils mit den extrakochlearen Empfangselektroden (E⁺, E⁻, 7a) verbunden sind, um repräsentative Signale der neuronalen Aktivität des Hörsystems ausgehend von den durch die extrakochlearen Empfangselektroden erfassten elektrischen Signalen zu erzeugen, **dadurch gekennzeichnet, dass** die extrakochlearen Empfangselektroden (E⁺, E⁻, 7a) auf einem nachgiebigen Träger (30) um ein Gehäuse (2) herum verteilt sind, welches das Verstärkermodul (4) beinhaltet.

2. Vorrichtung nach Anspruch 1, in welchem das Verstärkermodul (4) einen Differentialverstärker (12) umfasst, dessen Eingänge mit den Sammelelektroden (E⁺, E⁻, 7a) verbunden sind und dessen Masse mit einer Referenz-Sammelelektrode (RR₁, RR₂) verbunden ist.

3. Vorrichtung nach Anspruch 1 oder 2, in welcher die am Verstärkermodul (4) durch die Sammelelektroden (E⁺, E⁻ 7a) gebildeten elektrischen Signale eine Amplitude der Größenordnung von 0,1 bis 1 µV aufweisen.

4. Vorrichtung nach einem der Ansprüche 1 bis 3, in welcher die Sammelelektroden (E⁺, E⁻, 7a) in einem Abstand von höchstens 3 cm zueinander liegen.

5. Vorrichtung nach einem der Ansprüche 1 bis 4, in welcher das dichte Gehäuse (2), welches das Verstärkermodul (4) beinhaltet, auch einen Sender (5) für die kontaktlose Übertragung durch die Haut (10) von Funksignalen an eine externe Einheit (11) umfasst.

6. Vorrichtung nach einem der Ansprüche 1 bis 5, in welcher die Sammelelektroden (E⁺, E⁻, 7a) beiderseits des das Verstärkermodul (4) beinhaltenden Gehäuses (2) in einer Position im Wesentlichen diametral gegenüber liegend angeordnet sind und am Ende eines nachgiebigen Trägers montiert sind, der ihre optimale Positionierung ermöglicht.

7. Vorrichtung nach einem der Ansprüche 1 bis 6, in welcher die Sammelelektroden senkrecht zum Dipol der Stimulationsäquivalenz liegen.

8. Vorrichtung nach einem der Ansprüche 1 bis 7, in welcher eine Referenz-Sammelelektrode (RR₁) auf dem Gehäuse (2) befestigt ist, in welchem das Verstärkermodul (4) eingebaut ist.

9. Vorrichtung nach einem der Ansprüche 1 bis 8, in welcher eine Referenz-Sammelelektrode (RR₂, RS/RR₂) beweglich in Bezug zu dem Gehäuse (2) montiert ist, welches das Verstärkermodul (4) beinhaltet.

10. Vorrichtung nach einem der Ansprüche 1 bis 9, welche wenigstens zwei Referenz-Sammelelektroden (RR2, RS/RR) umfasst, die mit einem Schalter (19) verbunden sind.

11. Vorrichtung nach einem der vorhergehenden Ansprüche, in welcher die Stimulationseinrichtung wenigstens eine Stimulationselektrode (13, 17) zum elektrischen Stimulieren des Hörsystems umfasst, oder in welcher die Stimulationseinrichtung wenigstens einen Vibrator (23) zum mechanischen Stimulieren des Hörsystems umfasst.

12. Vorrichtung nach Anspruch 11, in welcher die Stimulationselektrode (13) eine intrakochleare Elektrode ist.

13. Vorrichtung nach Anspruch 11, in welcher die Stimulationselektrode (13) eine extrakochleare Elektrode ist.

14. Vorrichtung nach einem der Ansprüche 1 bis 13 mit wenigstens einer Referenz-Sammelelektrode (RR₂), die mit wenigstens einer Referenz-Stimulationselektrode (RS) gekoppelt ist.

15. Vorrichtung nach einem der vorhergehenden Ansprüche, mit ferner wenigstens einem in das Gehäuse (2) integrierten Multiplexer (8) und in welchem wenigstens ein Paar Sammelelektroden (E⁺, E⁻, 7a) mit dem Verstärkermodul (4) mithilfe des wenigstens einen Multiplexers (8) verbunden ist.
